Europäisches Patentamt

European Patent Office

Office européen des brevets

(11) Veröffentlichungsnummer: 0 056 493

A1

## (12) EUROPÄISCHE PATENTANMELDUNG

(21) Anmeldenummer: 81110804.2

(22) Anmeldetag: 28.12.81

(51) Int. Cl.³: **A 61 B 10/00**

(30) Priorität: 15.01.81 DE 3101109

(43) Veröffentlichungstag der Anmeldung:
28.07.82 Patentblatt 82/30

(84) Benannte Vertragsstaaten:
AT FR GB IT SE

(71) Anmelder: **von Zeppelin, Dieter**
**Wittelsbacherstrasse 20**
**D-8000 München 5(DE)**

(72) Erfinder: **von Zeppelin, Dieter**
**Wittelsbacherstrasse 20**
**D-8000 München 5(DE)**

(74) Vertreter: **Patentanwälte Dipl.-Ing. Klaus Behn**
**Dipl.-Phys. Robert Münzhuber**
**Widenmayerstrasse 6**
**D-8000 München 22(DE)**

(54) Gynäkologisches Abstrich-Instrument.

(57) Gynäkologisches Abstrich-Instrument für die Früherkennung von Krebszellen am Muttermund. Es besteht aus einer an einem Haltestab (10) drehbar gelagerten Aufnahmerolle (14) mit geschlossener Oberflächenstruktur. Die Oberfläche (14b) der Rolle (14) ist vorzugsweise um einen Winkel (α) von etwa 105° gegen die Längsachse des Haltestabes geneigt. Die Aufnahmerolle besteht aus einem Material mit adhäsiver Oberfläche, vorzugsweise aus Silikon-Latex.

Fig. 1

GYNÄKOLOGISCHES ABSTRICH-INSTRUMENT

Die Erfindung betrifft ein gynäkologisches Abstrich-Instrument
für die Früherkennung von Krebszellen am Muttermund mit einem
an einem Haltestab angeordneten, rotationssymmetrisch ausgebildeten Probennehmer.

Für einen Abstrich am Muttermund werden bisher Schaber oder
Wattestäbchen verwendet. Mit den Schabern kann eine verhältnismäßig große Menge an Zellmaterial abgenommen werden. Das Aufbringen des Zellmaterials auf einen Objektträger für die mikroskopische Untersuchung ist aber sehr umständlich, wenn eine
sichere Diagnose gewährleistet sein soll. Die Wattestäbchen
haben den Nachteil, daß das Zellmaterial tief in die Watte
eindringen kann, so daß beim Ausstreichen auf einen Objekttäger gegebenenfalls nicht alles Zellmaterial auf den Objekttäger übertragen wird. Auch kann es beim Aufstreichen zu Überlagerungen von Zellmaterial kommen. Dies führt zu Fehldiagnosen.
Die Treffsicherheit solcher zythologischer Untersuchungen dürfte deshalb bei nur etwa 40% liegen.

Es ist auch ein gynäkologisches Abstrich-Instrument der eingangs genannten Art bekannt (DE-OS 2 135 477), bei welchem
der aus porösem Material bestehende Probennehmer fest mit dem
Haltestab verbunden ist. Zur Entnahme von Zellproben wird der
Haltestab und damit auch der fest mit ihm verbundene Probennehmer gedreht und gegen das Gewebe abgerollt und abgezogen, so
daß Zellmaterial in die Poren des Materials eingerieben wird,
Die Handhabung dieses bekannten Probennehmers ist schwierig.
Außerdem ist es nicht einfach, den Probennehmer vollständig
auf einem Objektträger abzurollen, weil bei einem Abrollvorgang an sich eine Kreisbahn entsteht. Es ist deshalb nicht
sichergestellt, daß tatsächlich Zellmaterial von allen Teilen
des Muttermundes abgenommen wird und daß das abgenommene Zellmaterial auch wirklich vollständig auf den Objektträger aufgebracht wird. Das gleiche gilt auch in bezug auf ein weiteres
bekanntes Abstrich-Instrument in Form einer Bürste (GB-PS 2 010 084), das zur Abnahme von Zellmaterial ebenfalls insgesamt gedreht werden muß. Auch hier ist das Aufbringen des Zellmaterials auf einen Objektträger problematisch. Aus den genannten Gründen ist das Ergebnis von zytologischen Untersuchungen mit Hilfe der bekannten Instrumente unsicher.

Der Erfindung liegt die Aufgabe zugrunde, ein gynäkologisches Abstrich-Instrument der eingangs genannten Art zu schaffen, das bei einfacher Anwendung eine sehr sichere Diagnose ermöglicht. Dies wird erfindungsgemäß dadurch erreicht, daß der Probennehmer eine auf dem Haltestab frei drehbar gelagerte zylindrische Rolle aus einem Material mit geschlossener Oberflächenstruktur ist. Durch die drehbar gelagerte zylindrische Rolle wird erreicht, daß diese sich frei am Muttermund abrollen kann, so daß mit Sicherheit Zellmaterial von allen Stellen abgenommen wird. Durch die Verwendung von Material mit geschlossener Oberflächenstruktur wird ferner erreicht, daß alles abgenommene Zellmaterial an der Rollenoberfläche haftet, so daß es leicht auf einem Objektträger abgerollt werden kann, wobei die zylindrische Form der Rolle diesen Abrollvorgang auf dem Objektträger sehr erleichtert.

Zweckmäßig bildet die Rolle mit der Längsachse des Haltestabes einen stumpfen Winkel, der vorzugsweise etwa 105° beträgt.

Der Haltestab ist vorzugsweise an seinem freien Ende V-förmig abgewinkelt, wobei die Aufnahmerolle auf dem freien Schenkel der Abwinkelung gelagert ist. Dabei ist die Aufnahmerolle auf dem Haltestab zweckmäßig so gelagert, daß ihr äußeres Ende bis etwa an die Längsachse des Haltestabes reicht.

Die Aufnahmerolle besteht vorzugsweise aus einem Material mit adhäsiver Oberfläche, wie insbesondere Silikon-Latex. Sie ist zweckmäßig auf einem besonderen Lagerkörper aufgebracht.

Um eine sichere Lagerung und andererseits ein leichtes Aufbringen und Abnehmen der Aufnahmerolle zu gewährleisten, ist der Haltestab zweckmäßig mit wenigstens einem die Aufnahmerolle in ihrer Arbeitslage gegen eine Längsverschiebung verriegelnden Anschlag versehen, wobei die Aufnahmerolle und gegebenenfalls der Lagerkörper federnd ausgebildet sind, so daß sie
über einen am Ende des Haltestabes angebrachten Anschlag bewegbar sind.

Das erfindungsgemäße Instrument ermöglicht eine sehr einfache
Aufnahme von Zellmaterial von dem gesamten zu untersuchenden
Bereich des Muttermundes, indem durch Drehen des Haltestabes
die Aufnahmerolle über diesen Bereich abgerollt wird, wodurch
das Zellmaterial auf der Oberfläche der Rolle haftet. In ebenfalls sehr einfacher Weise kann das gesamte Zellmaterial durch
Abrollen auf einen Objektträger aufgebracht werden, wobei das
Material gleichmäßig und übersichtlich verteilt wird, so daß
es zu keinen Überlagerungen kommt. Damit ist eine sehr sichere
Diagnose ermöglicht.

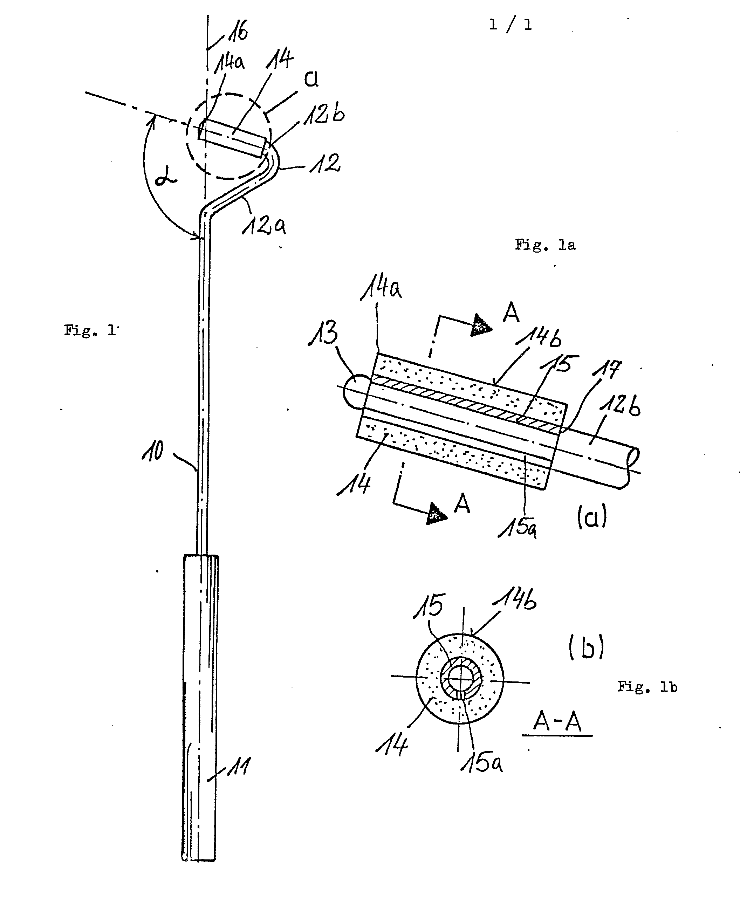

Die Erfindung ist im folgenden anhand der Zeichnung an einem
Ausführungsbeispiel näher erläutert. Die Zeichnung zeigt neben
einer Seitenansicht des erfindungsgemäßen Instruments eine
vergrößerte Einzeldarstellung (a) der Aufnahmerolle sowie eine
Schnittdarstellung (b) entlang der Linie A-A in (a).

Mit 10 ist der Haltestab bezeichnet, der an seinem rückwärtigen
Ende mit einer Handhabe 11 versehen ist. Der Haltestab 10 ist
an seinem vorderen Ende mit einer V-förmigen Abwinkelung 12
versehen, die aus den beiden Schenkeln 12a und 12b besteht.

Die Aufnahmerolle ist mit 14 bezeichnet. Sie besteht aus einem Material mit adhäsiver Oberfläche 14b, vorzugsweise aus
Silikon-Latex. Sie ist auf dem äußeren Schenkel 12b der aus
den Schenkeln 12a und 12b bestehenden Abwinkelung 12 drehbar
gelagert. Zweckmäßig ist die Rolle 14 auf einem besonderen Lagerkörper 15 aufgebracht, der auf dem entsprechenden Lagerteil
des Schenkels 12b eine leichte Drehung der Aufnahmerolle 14 gewährleistet. Der Schenkel 12b ist zweckmäßig an seinem freien
Ende, auf dem der Lagerkörper 15 gelagert ist, mit verringertem Durchmesser ausgebildet. Der Schenkel 12b bildet so bei 17
einen eine axiale Verschiebung der Rolle 14 verhindernden Anschlag. Am freien Ende ist der Schenkel 12b mit einer kugelförmigen Verdickung 13 versehen, welche die Rolle in ihrer Lage hält. Der Lagerkörper 15 ist beispielsweise mit einem Schlitz 15a versehen, der ein Aufweiten des Lagerkörpers 15 so weit ermöglicht, daß die Aufnahmerolle 14 mit dem Lagerkörper über die Verdickung 13 aufgebracht oder abgenommen werden kann.

Die Abwinkelung 12 des Haltestabes 10 ist vorzugsweise so geformt, daß die aufgebrachte Aufnahmerolle 14 mit ihrer äußeren Kante 14a auf der Längsachse des Haltestabes 10 liegt.

Zur Abnahme des Fellmaterials am Muttermund wird die Aufnahmerolle 14 mit Hilfe des Haltestabes 10 am Muttermund angelegt, und es wird die Handhabe derart gedreht, daß sich die Aufnahmerolle 14 über den gesamten Muttermund abrollt. Danach wird die Aufnahmerolle 14 auf einem Objektträger abgerollt. Eine gleichmäßige und übersichtliche Verteilung des Zellmaterials auf dem Objektträger ist dadurch gewährleistet.

Der Haltestab 10 mit Handhabe 11 ist zweckmäßig aus nicht rostendem Stahl hergestellt. Die Aufnahmerolle wird zur Durchführung eines Abstriches auf den Haltestab 10 aufgesteckt und nach Durchführung der Untersuchung und nach Abrollen auf einen Objektträger weggeworfen. Selbstverständlich ist es auch möglich, das ganze Instrument als Einmalartikel zu fertigen.

PATENTANSPRÜCHE

1.     Gynäkologisches Abstrich-Instrument für die Früherkennung von Krebszellen am Muttermund mit einem an einem Haltestab angeordneten, rotationssymmetrisch ausgebildeten Probennehmer, dadurch gekennzeichnet, daß der Probennehmer eine auf dem Haltestab (10) frei drehbar gelagerte zylindrische Rolle (14) aus einem Material mit geschlossener Oberflächenstruktur ist.

2.     Gynäkologisches Abstrich-Instrument nach Anspruch 1, dadurch gekennzeichnet, daß die Rolle (14) mit der Längsachse des Haltestabes (10) einen stumpfen Winkel ($\alpha$) bildet.

3.     Gynäkologisches Abstrich-Instrument nach Anspruch 1, dadurch gekennzeichnet, daß der Winkel ($\alpha$) etwa 105° beträgt.

4.     Gynäkologisches Abstrich-Instrument nach einem der Ansprüche 1 bis 3, dadurch gekennzeichnet, daß der Haltestab (10) an seinem freien Ende V-förmig abgewinkelt ist und daß die Aufnahmerolle (14) auf dem freien Schenkel (12b) der Abwinkelung (12) gelagert ist.

5.     Gynäkologisches Abstrich-Instrument nach einem der vorhergehenden Ansprüche, dadurch gekennzeichnet, daß die Aufnahmerolle (14) auf dem Haltestab (10) so gelagert ist, daß ihr äusseres Ende (14a) bis etwa an die Längsachse (16) des Haltestabes (10) reicht.

6.     Gynäkologisches Abstrich-Instrument nach einem der vorhergehenden Ansprüche, dadurch gekennzeichnet, daß die Aufnahmerolle (14) aus einem Material mit adhäsiver Oberfläche, wie insbesondere Silikon-Latex, besteht.

7.     Gynäkologisches Abstrich-Instrument nach einem der vorhergehenden Ansprüche, dadurch gekennzeichnet, daß die Aufnahmerolle (14) auf einem besonderen Lagerkörper (15) aufgebracht ist.

8.  Gynäkologisches Abstrich-Instrument nach einem der vorhergehenden Ansprüche, dadurch gekennzeichnet, daß der Haltestab (10) mit wenigstens einem die Aufnahmerolle (14) in ihrer Arbeitslage gegen eine Längsverschiebung verriegelnden Anschlag (13,17) versehen ist.

Fig. 1

16
14a 14 a
12b
12
12a

α

10

11

Fig. 1a

13 14a
A
14b
15 17
12b

A
14

15a (a)

15 14b

(b)

Fig. 1b

14
15a

A-A

0056493

Nummer der Anmeldung

**Europäisches Patentamt** · **EUROPÄISCHER RECHERCHENBERICHT** · EP 81 11 0804

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 4 227 537 (SUCIU) | 1 |
| A | GB - A - 1 378 393 (ANTONIDES)<br>* Seite 3, Zeilen 4-9; Figuren * | 1,6 |
| | -- | |
| A | US - A - 3 485 236 (FROST) | 1 |
| DA | FR - A - 2 103 054 (CURTIS)<br>& DE - A - 2 135 477 | 1 |
| | -- | |
| DA | US - A - 4 127 113 (NOLLAN)<br>& GB - A - 2 010 084 | 1 |
| | ---- | |

**KLASSIFIKATION DER ANMELDUNG (Int. Cl.³)**

A 61 B 10/00

**RECHERCHIERTE SACHGEBIETE (Int. Cl.³)**

A 61 B

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 16-04-1982 | STEENBAKKER |

EPA form 1503.1  06.78